# EUROPEAN PATENT APPLICATION

(11) **EP 2 682 884 A1**
(43) Date of publication of application: **08.01.2014**
(21) Application number: 13003372.3
(22) Date of filing: 03.07.2013
(51) Int. Cl.: G06F 19/00

(54) **Apparatus for securely transferring, sharing and storing of medical images**

(30) Priority: 05.07.2012 BE 201200466; 20.11.2012 US 201261728543 P
(71) Applicant: Perennity SPRL-BVBA, 1090 Bruxelles (BE)
(72) Inventor: Janssens, Laurent, 1700 Dilbeek (BE)
(74) Representative: Theunis, Patrick

(57) **Abstract**

The invention relates to an apparatus for receiving, storing, sharing and transmitting digital medical image data, comprising a processor, a hard drive, and connectivity means for communicating with a local or global network. The processor comprises means for hosting a server supporting receipt, compression, encryption and data integrity check of the medical image data received from medical modalities, storing of the medical image data on the hard drive of the apparatus, providing access to the apparatus' web based administration and management interface and supporting the query and retrieval of medical image data stored in the apparatus from any modality.

## Description

### Technical field of the invention:

The present invention relates to a small portable, hand sized "All-in-One" apparatus for securely transferring, sharing and storing of medical images using Internet even with a limited bandwidth. In the following description, this apparatus is referred to as "Apparatus".

The invention also relates to a web portal for accessing the medical images transferred by the apparatus according to the present invention.

### Description of the prior art

The Apparatus and solutions that the present inventors have developed up to now are based on the use of the well-known DICOM Standard and are using media like CD, DVD and Bluray for the distribution, sharing and archiving of medical images. The best example is the "Patient CD", replacing films, used to transfer and share medical images between clinics or radiologists and their patients, referral physicians, specialists and hospitals. DICOM is the acronym for Digital Imaging and Communications in Medicine and is a standard known for the person skilled in the art that describes how medical image data should be handled, stored, distributed or transmitted and printed.

Today, in Europe and North America, medical image distribution and archiving can be done using Internet due to the significant bandwidth increase, a lowering of the infrastructure costs and network security improvements (VPN, private networks, ...). As a result, it makes sense that hospitals may want to replace Patient CDs by a solution to exchange and transfer medical images using the Internet. It makes also sense to provide access for Doctors and even Patients to review their medical images, including the diagnostic report, using a secure WEB-based portal.

Today, some "Data Centers" are offering secure hosting solutions to archive and share medical images. However, in the medical imaging business, some doctors have some reservations to transfer data across the Internet. Also, even if the Internet bandwidth is continuously increasing, the resolution and as a result hereof the size of the digital medical images are also dramatically increasing. Last but not least, the Internet infrastructure in most emerging countries does not permit to consider using Internet to transfer digital medical images because of the limited bandwidth and reliability of the Internet network. It is obvious that the internet infrastructure will improve in the coming years but not right away.

In order to solve the abovementioned problems the present inventors have developed an Apparatus and web portal as set forth in the appended claims.

### Description of the invention.

Basically, the Apparatus according to the present invention comprises a LINUX based mini-PC with local storage capacity. The Apparatus has been designed to transfer, distribute and archive medical images using the Internet. The Apparatus has been engineered to cope with limited bandwidth Internet connections and is able to manage data flow interruptions.

The Apparatus according to the invention comprises an advanced rule-based router for medical images including hardware and software. According to a preferred embodiment, it includes a high capacity buffer/storage. Thanks to this buffer, the images can be transferred using Internet even with limited bandwidth, interrupted and/or disruptive connections.

The Apparatus manages compression, encryption and data integrity check of the medical images transmitted across the Internet.

Optionally, those images can be reviewed by referring physicians and even patients using a WEB portal.

According to a preferred embodiment, the Apparatus is also capable of replicating its content to a local network storage (e.g. a NAS with a RAID controller and several hard drives) and/or to an external secure "Data Center". Different options are available. The first is based on the Amazon S3 services (see http://aws.amazon.com) providing a worldwide network of Internet services like data storage hosting and virtual servers. The second option is to use a FTPS server (File Transfer Protocol Secured) or SFTP server (SSH File Transfer Protocol) hosted on the Apparatus itself, by a third-party Internet Service Provider or even by a hospital centralizing the medical images supplied by external clinics or mobile units.

The Apparatus according to the present invention is capable of performing one or more of the following functions, and as such can be used for a wide variety of applications in the medical field :
- Transferring images from Imaging center(s) or Radiology departments to Diagnostic center(s);
- Transferring images from several "satellite" clinics or imaging centers to a central hospital for diagnose;
- Transferring images from a central hospital or diagnostic center to Radiologist(s) working from a remote facility or home;
- Storing DICOM images as a local archive in an imaging center or small clinic;
- Archiving DICOM images to an external Secure Data Center;
- Sending DICOM images "instantly" from Mobile Radiology equipment travelling to distant cities and villages (e.g. trucks equipped with a CT/MRI scanner or other radiology equipment) using wireless 3G/4G connectivity;
- Publishing DICOM images and diagnostic reports using a WEB portal for referring physician and potentially patients;
- Polyclinics wishing to send medical images to referring physicians;
- Sharing a CD/DVD robot between different facilities from a central location;
- Transferring CT images to a shared 3D printer (e.g. dental modelling).

### Installation and maintenance of the Apparatus :

The installation of the Apparatus of the present invention is extremely simple: prior to shipment to the customer, the Apparatus not only has been preinstalled but also preconfigured based on technical data provided by the end-user (network infrastructure, radiology equipment). The transfer parameters (Amazon or FTPS/SFTP) are also preconfigured. The pre-installation (hardware assembling, operating system installation, installation of our software modules) is performed at manufacturer's facilities or by its sales/technical partners. The end-user needs only to unpack the Apparatus, plug the power and network cable and he will be ready to go. Obviously, the only requirement is an Internet connection through the LAN (Local Area Network) or a 3G/4G USB module, available as an option.

Last but not least, the maintenance of the Apparatus is really easy: an encrypted backup of the database and the configuration parameters are automatically saved to an USB stick (provided with the Apparatus). In case of a failure, the customer needs only to insert that USB key in a new Apparatus (provided to the customer under the terms of a maintenance contract). Both database and parameters will automatically be restored. The data (i.e. the images) stored on the local hard drive will also be restored from the local NAS or the external Data Center. It is definitely a great benefit for medical institutions that do not have skilled IT people on site. No need also for resellers and distributors to send a technician or engineer on-site which can be expensive and time consuming in large countries.

### Fields of application of the Apparatus :

The Apparatus has been designed to fit all requirements, starting from small clinics with a limited Internet infrastructure and budget up to the largest medical institutions wanting to exchange images with their subcontractors (e.g. acquisition centers, private radiologist). The Apparatus suits also to referring physician, doctors and patients who want to review their images. The Apparatus is appropriate for Europe and North America having fast Internet connexions as well as for emerging countries having limited Internet bandwidth not allowing a reliable transfer of large files containing medical images.

### Detailed description of the invention :

### Hardware Design

The hardware design of the Apparatus according to the present invention is based on a personal computer manufactured by Foxconn and marketed under the brand name "NanoPC".

So the Apparatus according to the invention comprises the following features comprises in such PC : an Intel ATOM processor, a frame for a 2.5" hard drive and all required connectivity: USB 3 ports, memory card reader, Gigabit Ethernet, Wi-Fi interface with built-in antenna, HDMI and VGA video output.

To this configuration, the present inventors have added 2GB of RAM which is more than sufficient for performing the functions and applications required under the present invention and the LINUX operating system. The NanoPC is only 19cm (7,5") wide, 13 cm (5") deep and 3cm (1") high. The noiseless radial fan allows air circulation from the left to the right side.

The NanoPC emits limited heat which considerably preserves the lifetime of the internal hard drive. The temperature does not exceed 40° Celsius (104° Fahrenheit) in a 25° Celsius (77° Fahrenheit) room.

The Apparatus according to the invention only consumes less than 20W which makes it very environment friendly.

The high speed heavy duty hard drive is intended to be used for audio and video streaming which dramatically increases the data transfer while extending its lifetime.

A mobile internet module (2G, 3G and 4G) is also available as an option. It allows transferring images using a wireless connection which is especially useful for mobile radiology trucks or clinics located in place not having a wired Internet connection.

The Apparatus according to the invention is inserted in a transparent acrylic U form cover with a white painted inside. This cover hides the front connectors (USB port, memory card reader, ...) that are not used. Only a round hole in the front provides access to the power button. A pen must be inserted in that hole to push on the button which is on purpose to avoid accidental power off. The Apparatus is supposed to be on 24h by 24h.

In case of a power failure, the Apparatus will reboot automatically when the power is restored.

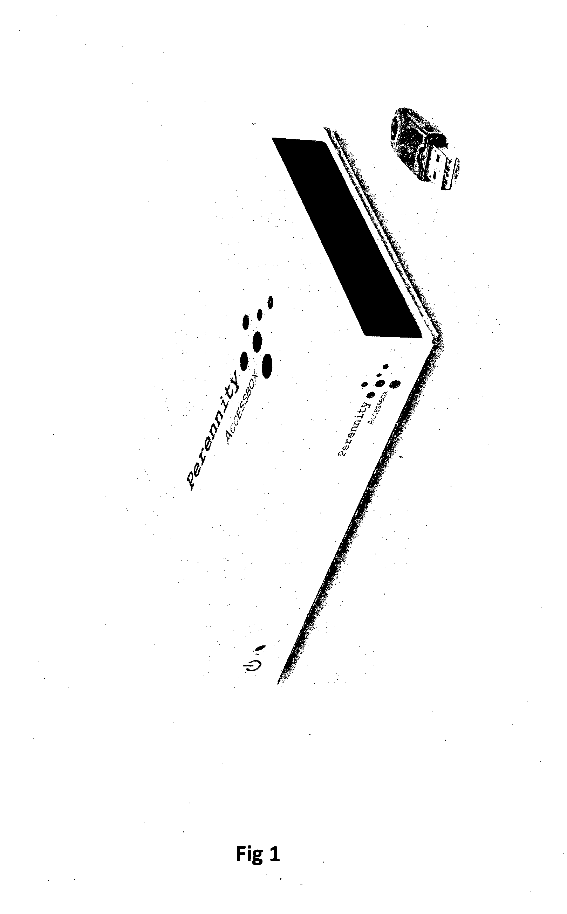

Reference is made to figure 1 for illustrative purposes.

The Apparatus according to the invention further comprises an external USB storage key. The key contains:
- The software activation key
- The configuration of the different software modules
- A daily backup of the local MySQL database
- A Network file "template" allowing to change the IP settings of the Apparatus.

### Extended configuration of the Apparatus.

In the case of a large number of institutions having to send DICOM images to a central location (e.g. 20 imaging centers to a central diagnostic center), the performance of the Apparatus according to the invention located at the central place may be too slow. For such a situation, an extended configuration of the Apparatus appears to be the right solution. Basically, such can be offered in the form of a Virtual Server (e.g. VMWare) image that can be installed on any server running exactly the same functionality as the Apparatus of the invention but offering more power, responsiveness and increased data storage capacity.

### Software architecture of the Apparatus according to the invention

### - Operating system & environment

The Apparatus is Ubuntu LINUX based. Only the LINUX kernel is kept to improve the overall performance. The LINUX graphical user interface is not installed.

All software modules of the Apparatus are JAVA based and de facto support most operating systems like LINUX (like the Apparatus itself) and Windows (for the WEB portal).

All software modules are using an open standard database engine (e.g. MySQL) using JDBC (JAVA Database Connectivity).

The Apparatus also hosts an open standard WEB server (e.g. Apache Tomcat). Its main purpose is to provide access to the Apparatus' WEB based management administration interface.

### - DICOM Server

The main module is a genuine DICOM server that runs as a LINUX daemon. It supports:
- DICOM Store, i.e. C-STORE (e.g. DICOM modalities sending images to the Apparatus.)
- DICOM Query/Retrieve, i.e. C-FIND & C-MOVE (e.g. Query and Retrieve DICOM images stored in the Apparatus from a DICOM modality like a Diagnostic Workstation)

The DICOM Server can be configured to automatically forward received DICOM images (using C-MOVE) to a different modality (e.g. PACS, Diagnostic Workstation, ...).

According to a preferred embodiment of the invention, the DICOM Server also manages replication of the DICOM images stored in the internal hard drive to a NAS (Network Attached Storage), DAS (Direct Attached Storage) and CAS (Content Addressed Storage). This "secondary" storage device is also used to extend the capacity of the internal HDD. According to a further preferred embodiment of the invention, when the Apparatus, in particular its hard disk is running out of space, the oldest images will automatically be deleted (FIFO) while a copy will remain available from the secondary storage.

### - DICOM Router

Depending on some criteria (e.g. the type of modality the images have been sent from), the DICOM router module may apply some changes to the properties of the images (e.g. anonymize the metadata of a series of images) and/or route the images to a defined destination (e.g. a diagnostic workstation).

Here are the different criteria that may apply:
- Value(s) and/or presence of a DICOM tag(s)
- Calling AET (i.e. the modality where the images are sent from)
- Called AET (i.e. the modality where the images are sent to)

### Multiple criteria can be combined.

Here are the different actions the module may perform:
- Change the value of DICOM tag(s)
- Add/Remove DICOM tag(s)
- Route the image to a defined destination
- Change the Transfer Syntax (i.e. compress/decompress the images)

### Multiple actions can be combined.

### - Transfer Module

This module is responsible to manage the transfer of DICOM images between two or more Apparatuses. The Transfer Module is the gateway between the DICOM network and the transfer supported technologies i.e. Amazon S3, any FTPS/SFTP server or other proprietary file transfer technologies.

Optionally a SFTP (Secure File Transfer Protocol) server can be installed on the Apparatus itself allowing "peer-to-peer" secure transfer of DICOM images. Transfer is secured by a SSH tunnel encrypting the exchange of series of DICOM images using FTP servers hosted on the Apparatus.

### - HL-7 Server Module

This module is responsible for the reconciliation of the Patient data stored in images received by the Apparatus prior to forward them to a PACS and/or a Diagnostic Workstation.

### - Email notification module

As the Apparatus is a "black box" without any display, an email notification is available to warn users in case of an issue. The same module will send a daily report with the list of all the studies that have been sent and received. That report is sent as an encrypted PDF attachment to the email.

### Management interface of the Apparatus according to the invention

The Apparatus does not have the LINUX graphical user interface (GUI). This is done deliberately to optimize the overall performance of the hardware. Also, it is not supposed to be connected to a keyboard, a mouse and a monitor.

A WEB based remote user interface using HTML5/JQuery technologies hosted by an Apache Tomcat WEB server is directly installed on the Apparatus. HTML5/JQuery user interface makes it compatible with any WEB browser (Internet Explorer, Google Chrome, Safari, ...) on all operating systems.

Before accessing the WEB interface, a user name and password are required. Those can be saved in a cookie.

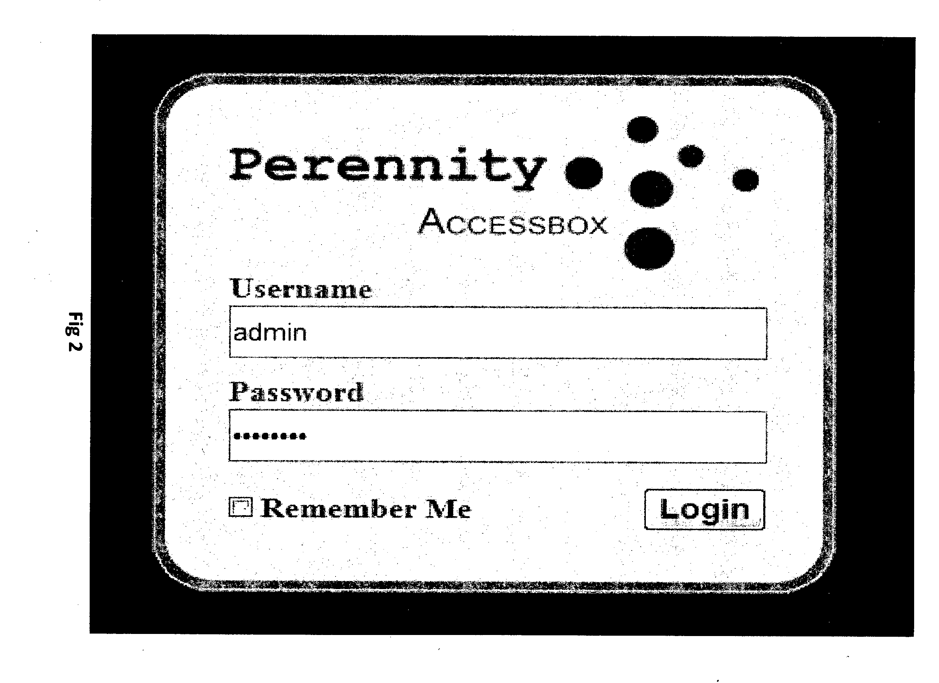

Reference is made to Figure 2 for illustrative purposes.

Like the Apparatus' hardware design, the WEB pages are simple and sleek.

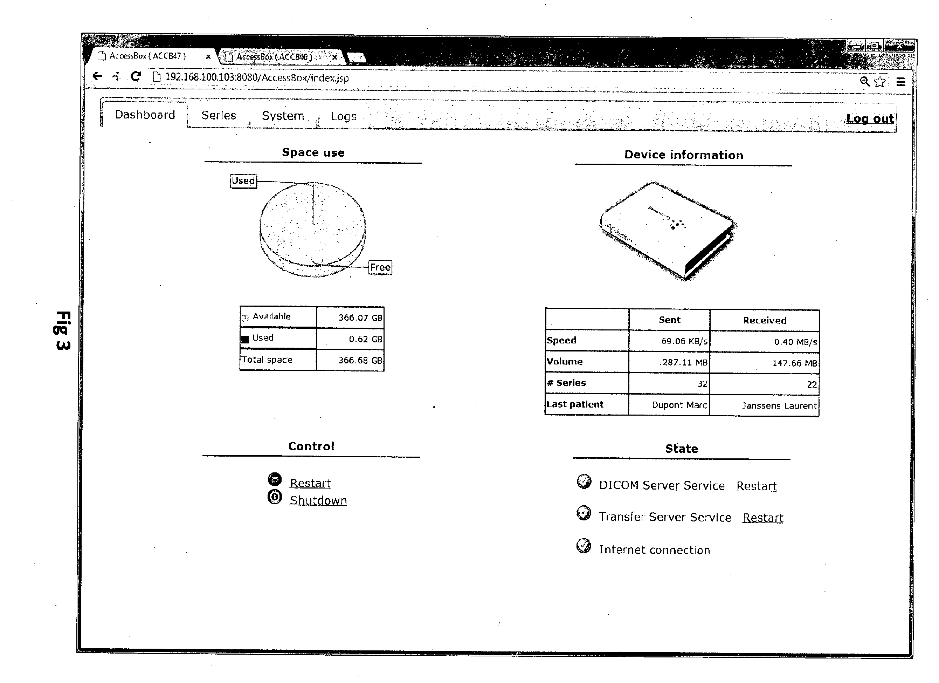

Reference is made to Figure 3 for illustrative purposes.
- Content of the Apparatus :
   The "Series" tab shows the list of the series of DICOM images that have been sent and received. It is possible to filter on the date (Today, Yesterday or All). There is also a convenient "Search" field that will filter the list of the series based on some keywords or parts of keywords.

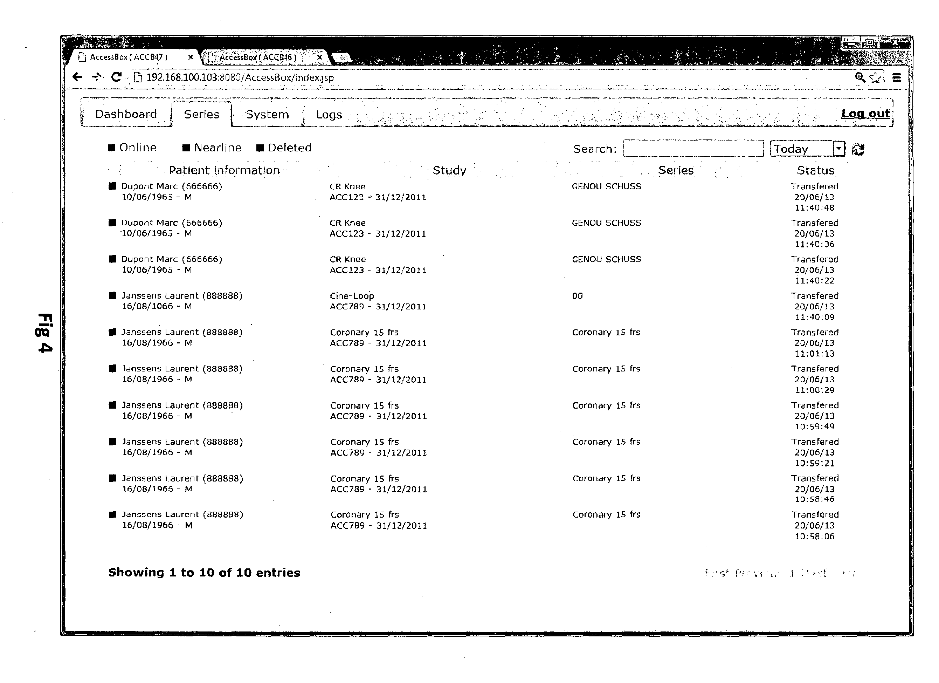

Reference is made to Figure 4 for illustrative purposes.

### - Multilingual user interface

The WEB interface is available is English, French, Dutch, Spanish, Brazilian Portuguese, Chinese, Russian, German, Polish and Italian. Our HTML5 interface has been designed to support any type of characters including Unicode 2-bytes.

### - System configuration interface

The WEB based system configuration interface allows to configure the network parameters (IP address, subnet mask, DNS, ...) and AETs (Application Entity Title) identifying all modalities (CT scanner, ultrasound, diagnostic workstation). When two or more Apparatuses have to communicate with each other, the workflow can also be configured in the WEB interface, each Apparatus being identified by its AET.

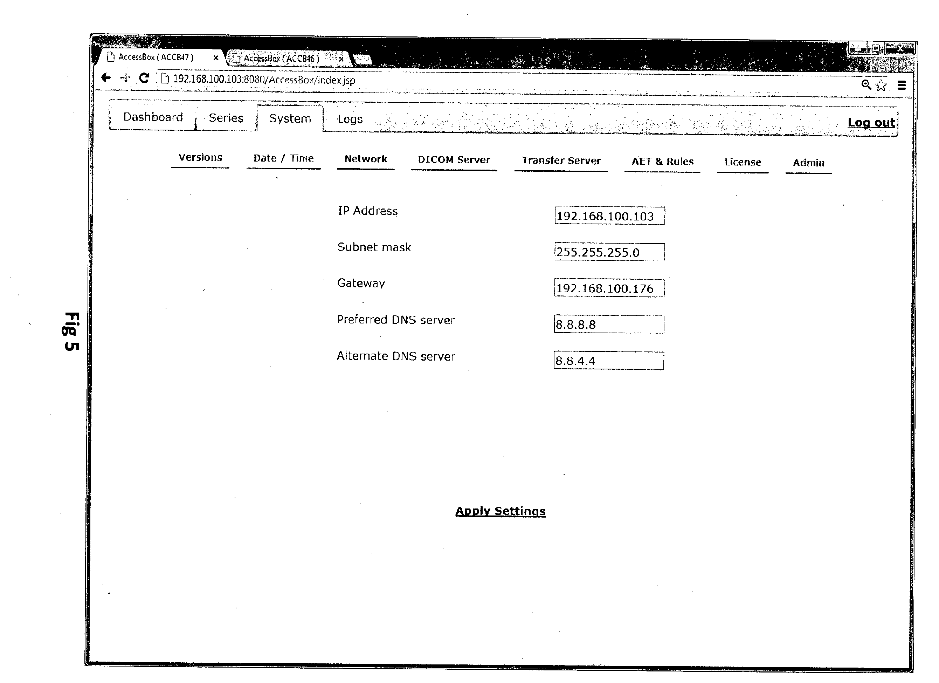

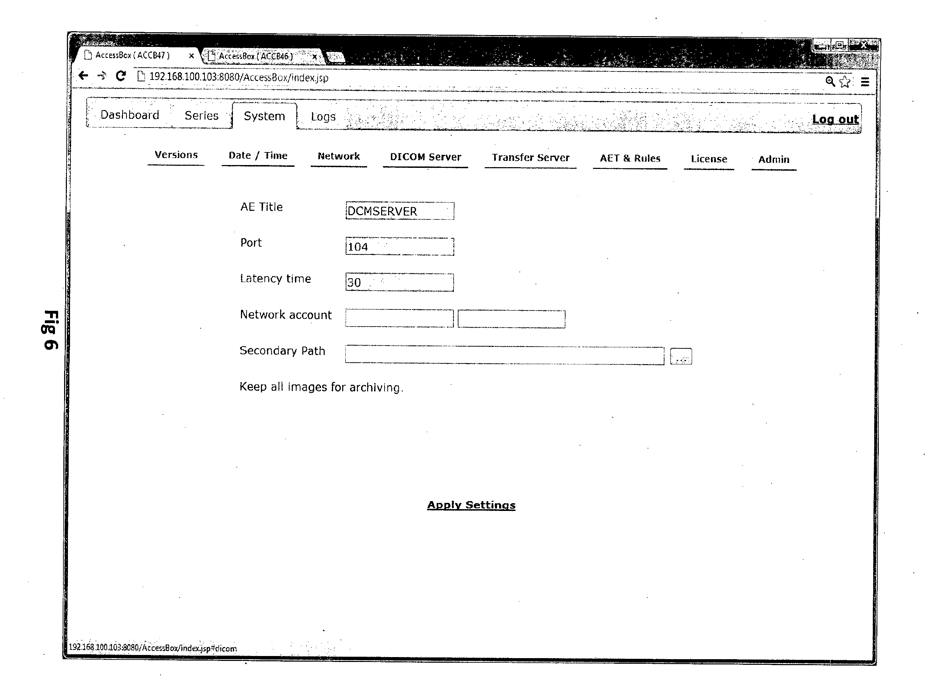

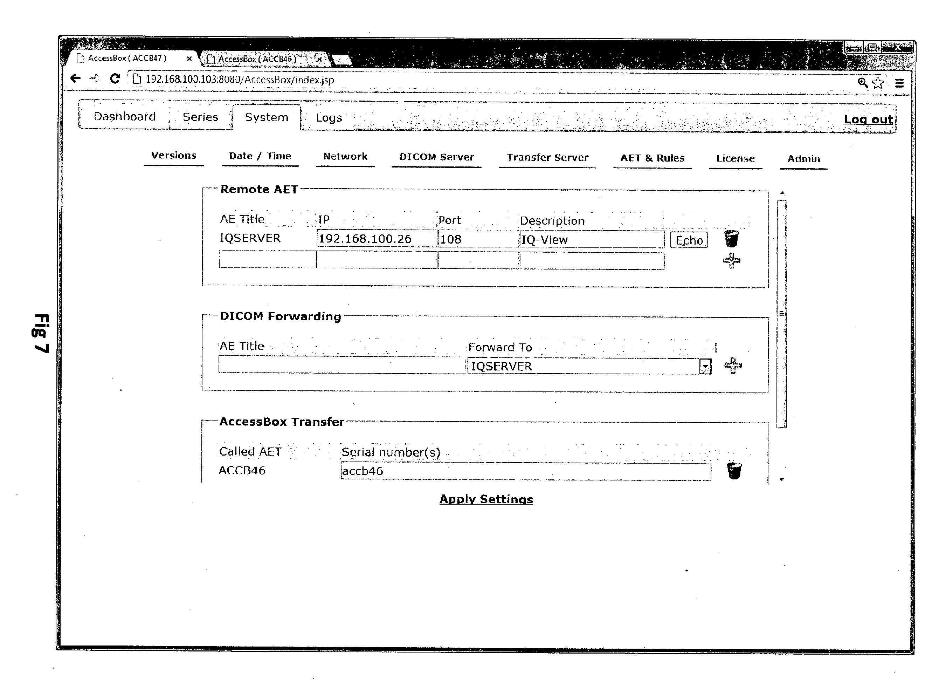

Reference is made to Figures 5, 6 and 7 for illustrative purposes.

It is also possible to generate activity reports and retrieve error logs. Preferably, one log file per day is created. Those files can be downloaded from the Apparatus for reference and/or analysis.

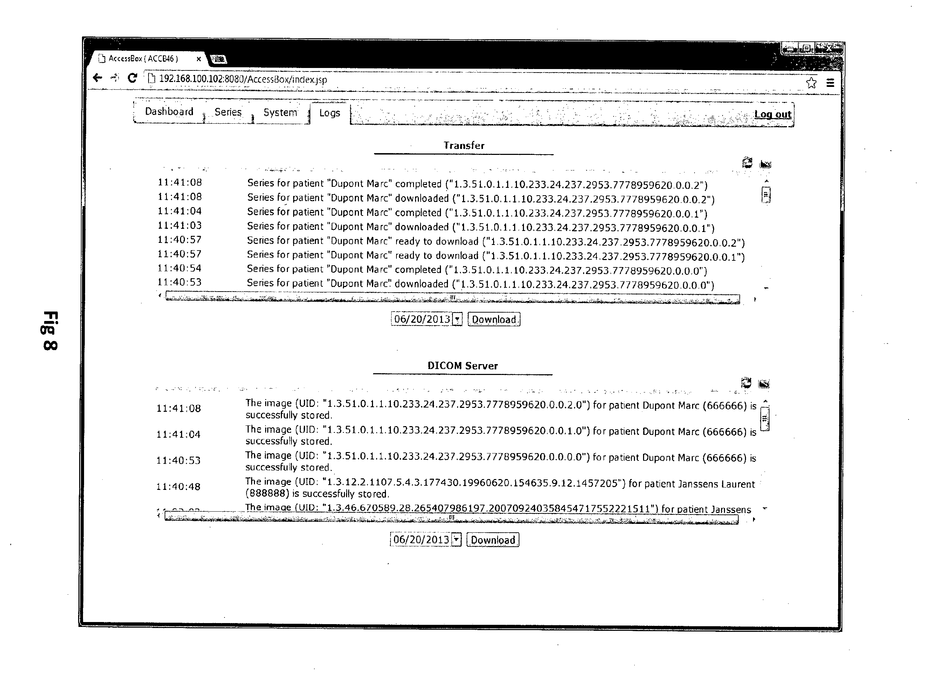

Reference is made to Figure 8 for illustrative purposes.

### - Advanced configuration interface

All system parameters of the Apparatus can be configured using the WEB based Webmin graphical interface. This tool lets you Apparatus configure a Proxy, manage third backup of the database and file system, update the operating system and much more.

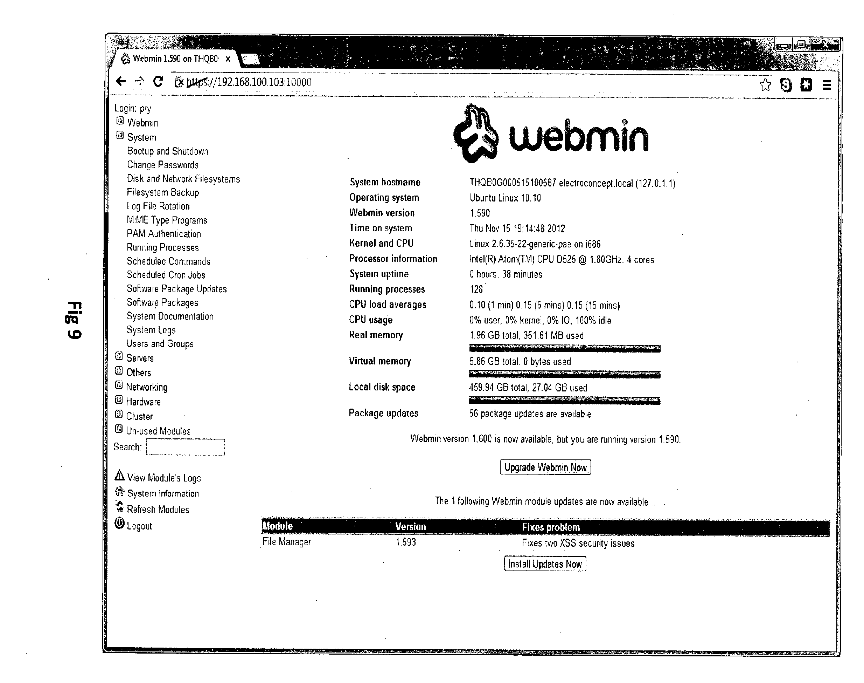

Reference is made to Figure 9 for illustrative purposes.

### Main processes

### - DICOM Image transfer between Apparatus.

The DICOM image transfer mechanism of the Apparatus preferably meets the following requirements: the workflow must be easy to use and implement, work with any kind of Internet connection (even slow and unstable), be highly secure - without the need of using VPN (Virtual Private Network) - slow, expensive and hard to implement -, leased lines or Private Cloud -, be cost effective and can be implemented in the entire world. The Apparatus preferably complies with local legislations related to storage, archiving and sharing of medical and patient data (e.g. HIPAA).

The first option is using the Amazon WEB Services (AWS) providing a powerful worldwide infrastructure to transfer files, host virtual servers and highly secured databases for a decent price. AWS (see aws.amazon.com) has Data Centers in the US, Brazil, Ireland, Singapore, Australia and Japan. The Apparatus may use the Amazon S3 platform to transfer DICOM images (e.g. from one Apparatus to another one) and Amazon EC2 for virtual servers (e.g. to host our WEB portal for referring physicians to review medical images from their patients online).

The second option uses a FTPS or SFTP server hosted by an Internet Service Provider (ISP) or directly in the Data Center of a hospital.

The third option is to host a FTP server on each Apparatus and exchanging series of DICOM images using a SSH secured communication tunnel (equivalent to SFTP). The main benefit is that Apparatuses can transfer DICOM image without the need of a third party Internet Service Provider hosting a FTPS or SFTP server. Also, DICOM images will be transferred directly between Apparatuses without needing a VPN connection (File transfer is much faster using FTP across SSH).

Amazon S3, FTPS and SFTP (local or remote) technologies offer the same level of security when transferring data from/to their servers. The communication is encrypted using SSL (Secured Socket Layer 256 bit with certificates). However, transferring images using a local FTPS/SFTP server guarantees that the exchanged data will be stored in the same country to comply with some local regulation.

### - Transfer Workflow

Definitions:
○ Bucket: it is a folder accessible from the root of a FTPS, SFTP (hosted on the Apparatus or any Data Center) or Amazon S3 account. This bucket may contain files that can be stored, renamed, moved or deleted by participating Apparatuses.
○ Order Buckets - Order Files: each Apparatus has a dedicated Order Bucket where "Order Files" will instruct the Apparatus what action it has to perform (e.g. download a series of DICOM images that are ready to be transferred). The name of an Order file contains the Series UID (unique identifier for a series of DICOM images, typically a long chain of digits separated by dots) preceded by the status of the order to be, being or been performed ("download." means that a series is ready to be downloaded, "ok." Means that a series has been fully downloaded).
○ Series Container: it is a single file which contains DICOM images that belong to the same series of images (e.g. the sagittal series of images from a CT scanner). When adding DICOM images to a Series Container, they are first compressed but without any loss of information (lossless compression). The algorithm used for the compression is ZIP. Optionally, Series Containers can be encrypted using the AES 256 bit algorithm.
○ Series Bucket: a group of Apparatuses that have to exchange DICOM images will share one single "Series Bucket" that will contain the "Series Containers" to be transferred.
○ AE Title (AET): the externally known name of a DICOM device or program, used to identify a DICOM application to other DICOM applications on the network. It is typically labelled with numbers and uppercase characters only. An AET is always associated with a unique combination of IP address and IP port.
○ Called AET: the destination AET i.e. the DICOM device or program receiving DICOM images or instructions.
○ Calling AET: the source/origin AET i.e. the DICOM device or program sending DICOM images or instructions.
○ Transfer Association (TA): it is the relationship between a Called AET defined on an Apparatus and the Order Bucket of the Apparatus the DICOM images have to be sent to.
○ Info file: a text file stored in the Series Bucket that provides information about a series of DICOM images including partial Patient information, the Called AET and Calling AET. There is one Info file per Series Container.

Workflow:
Here is an extended explanation about the workflow when an Apparatus sends one or more series of DICOM images to one or several Apparatuses.
   1. A Modality (Calling AET) sends DICOM images - using C-Store - to a local Apparatus specifying the target Apparatus based on its Transfer Association (TA) i.e. its Called AET.
   2. When getting the images, the DICOM Server of the local Apparatus will store the images on the local hard drive. Images are sorted per study date, study UID and series UID.
   3. The DICOM Server will apply a latency time (parameter) before sending the images, series by series to the Transfer Server, including the TA based on the
      Called AET specified by the Modality.
   4. The Transfer Server will compress the DICOM images creating one Series Container per series of images.
   5. Optionally the Series Container will be encrypted using AES 256 bit.
   6. Once the Series Container is ready, it will be transferred to the common Series Bucket belonging to the group of Apparatuses exchanging DICOM images. The Transfer Server cuts the Series Container in chunks of 5MB before transferring them to the Series Container. Once all chunks are uploaded, the Series Container will be consolidated in the Series Bucket.
   7. The Transfer Server creates and stores the Info File in the Series Bucket.
   8. The Transfer Server will create an Order file to instruct the remote Apparatus(es) that a new Series of images is ready to be downloaded. The Order file will be dropped in all Order Buckets belonging to the remote Apparatus(es) based on the TA defined by the Called AET provided by the source modality. The prefix of the Order file is "download." followed by the series UID of the set of images.
   9. The remote Apparatuses are scanning their own Order Buckets to check for the presence of a "download." Order file. If one is found, the remote Apparatus will download the Series Container, also in chunks of 5MB, from the Series Bucket based on the series UID contained in the Order file. If for any reason, the transfer of a chunk fails, the download of the next chunks in the sequence will start again to avoid the download of the entire Series Container again.
   10. Once downloaded, an integrity check of the Series Container will be performed before be decrypted and decompressed by the Transfer Server of the remote Apparatus. Then, it will send the images, one by one, to the DICOM Server of the remote Apparatus.
   11. Depending on the Called AET provided by the Info file associated to the Series Container and if forwarding rules have been defined in the DICOM Server, it will send the images to the Called AET.
   12. Once all images stored locally on the remote Apparatus and optionally forwarded to a different AET, the Transfer Server will rename the prefix of the Order file from "download." to "ok." to instruct the source Apparatus that the transfer is completed.

### - Encryption of the Apparatus hard drive

The DICOM images of an Apparatus are stored on an encrypted partition of the local hard drive. This partition is encrypted preferably using "Truecrypt", an open-source disk encryption software for Windows, Mac OS X and Linux. The encryption method requires a private key that is stored on the USB stick of the Apparatus. When booting an Apparatus, the private key is required to access the encrypted partition and de facto the USB key must be plugged. The private key used to encrypt the partition is encrypted using another secret key, the Master Key, that only one person in the Apparatus development team knows. A copy of that Master Key is kept at an escrow. To avoid any access to that Master Key on the Apparatus itself, that key is stored in a compiled batch program responsible to mount the encrypted partition. This batch program can only be compiled by the sole person having the Master Key.

If an Apparatus is defective and needs to be sent back to the manufacturer or repair facility, it should be returned without the USB stick. As result, nobody will be able to mount the encrypted partition and access the DICOM images.

### - Encryption and compression of the Series containers

Series of DICOM images are stored in compressed and encrypted containers prior to be uploaded to a FTPS/SFTP or Amazon S3 server. As result, no single person in the Data Center hosting the FTPS/SFTP server or at Amazon facilities will have access to the DICOM images stored in the encrypted containers.

The compression algorithm used by the Transfer module of the Apparatus is ZIP. ZIP compression is very efficient on DICOM images. Compression ratios range from 6:1 (e.g. mammography, CT, MRI) down to 2:1 (e.g. thorax). ZIP compression guarantees a 100% lossless compression.

The compression method includes an intrinsic data integrity mechanism. A CRC checksum is calculated when compressing the container. When decompressing the container a checksum is calculated and compared to the original one. If they do not match, the container is corrupted and an error will be reported. The container may be downloaded again.

### - Encryption of access keys

When using a FTPS server, a user name and password must be provided. A SFTP server requires a public key and secret key while Amazon S3 needs an "AccessKey" and "SecretKey" (Amazon terminology). All those parameters are stored in configuration files on the Apparatus and a backup copy is also stored on the USB stick. As those parameters are sensitive, they are all encrypted using the Master Key using the compiled batch program described above.

Containers are encrypted using the AES 256 bits which ensures the highest level of data protection. The secret key used to encrypt a container is generated randomly and stored in the Apparatus database. Before uploading the container to FTPS/SFTP/S3 to transfer it to a remote Apparatus, the random password is provided to it using its Order Bucket. The random secret password is also encrypted using the Master Key.

### - Apparatus Local Data Replication

The Apparatus is equipped with only one internal large capacity hard drive. Because of the very small size of the Apparatus, there is no extra physical space to put a second hard drive (e.g. building a RAID 1 mirror). The single Apparatus hard drive contains the medical images (on an encrypted dedicated partition. De facto, they are a critical data. Its content can be replicated to a secondary storage device like a NAS system (CIFS/NFS Network Attached Storage or iSCSI) or local external hard drive(s) via USB (Direct Attached Storage - DAS). Most of those NAS/DAS systems have an embedded RAID controller securing the files replicated across several disks. If one hard drive (or even two when using RAID 6) fails, no data will be lost.

Each time an Apparatus gets a file (i.e. a medical image), it is not only stored on its internal hard drive but also replicated to the NAS/DAS. As result, the Apparatus always retains two copies of each image. The database stores the path of all series of images to the local hard drive and the copy to the secondary storage.

When the local hard drive is full, oldest images will automatically be deleted (FIFO - First In First Out). In that case, only one copy will remain available from the NAS. The Apparatus keeps access to all images restored either from the local hard drive or from the NAS. When the NAS system is full, an additional unit can be added. Only the path to the new added NAS needs to be updated in the configuration of the Apparatus.

In case of a crash of the Apparatus hard drive, a swap unit will be provided. The database can be restored from the original USB stick. This database contains the list of the images that were stored on the hard disk before crashing and also the path to the copy of those files on the secondary storage device. As result, it is possible to recover the original content of the local hard drive.

### - Apparatus Off-Site Data Replication

The DICOM images exchanged between Apparatuses are transiting through external Series Bucket (FTPS/SFTP or Amazon S3 servers). The Series of images are stored and transferred using compressed and encrypted container (one container per series of images). To reduce the storage space on the FTPS server, we keep those container as it without uncompressing them. In case of a disaster causing the loss of all the data locally (i.e. where the Apparatus is located), the ZIP containers can be downloaded from the remote FTPS/SFTP/S3 server and be uncompressed by a new Apparatus locally.

The transfer of those containers is secured using SSL encryption.

### - Advanced routing of DICOM images

An Apparatus can automatically forward the received images to an other modality like a PACS or a Diagnostic Workstation. Automated Forwarding rules can be defined based on the Called AET and the TA (Transfer Association).

Received images can be also be handled by the embedded DICOM Router. Based on different criteria or combination of criteria (Calling AET, Called AET, Tag presence/value), images can be modified (Transfer Syntax, Tag value, ...) and/or forwarded to a defined AET.

### - Patient data reconciliation

The Apparatus includes a reconciliation process avoiding any inconsistency between the metadata of the transferred images (e.g. the patient ID) and the metadata of images stored in a PACS (Picture Archiving and Communication System) of the target hospital. The reconciliation process is managed either by the embedded HL-7 server interacting with a RIS (Radiology Information System) or HIS (Hospital Information System) querying patient information based on his name, birth date and sex. The DICOM Server can also perform a C-Find to query the PACS based on the same information. If the Patient information returned by the RIS, HIS or PACS is matching exactly with the one store in the DICOM images, they will automatically be forwarded to the defined AET (e.g. a PACS). If not, the tags that are not matching (e.g. the Patient ID is different), will be updated accordingly. The reconciliation process may require human intervention if no relationship can be made between the data contained in the images and the data queried from the RIS/HIS/PACS.

### - Controlled publishing of medical images to a WEB portal

The referring physician and sometimes the patients (this depends on the country) must have access to their medical images preferably using a WEB portal. In that case, it is obviously mandatory that patient have only access to their own images and the referring physicians to the images belonging to their own patients.

Our WEB portal is composed by two modules: the first is used to display the images and the second one is managing the access rights.

Our WEB portal is based on a Windows 2008 or Linux Server either hosted by Amazon (EC2 virtual server platform) or a Data Center. It runs Apache Tomcat as WEB server and any WADO compatible DICOM Web-based viewer (WEB application to display medical images in a WEB browser and having some basic tools like zooming, windowing and linear measurements).

DICOM images are rendered by the WADO module and sent as JPEG/PNG files to the client's WEB browser. Every time a new change is requested like zooming or change the windowing, the DICOM images will be rendered again and its JPEG/PNG representation will be sent back to the WEB client. This process makes the WEB portal extremely reactive and fast, even with slow Internet connections.

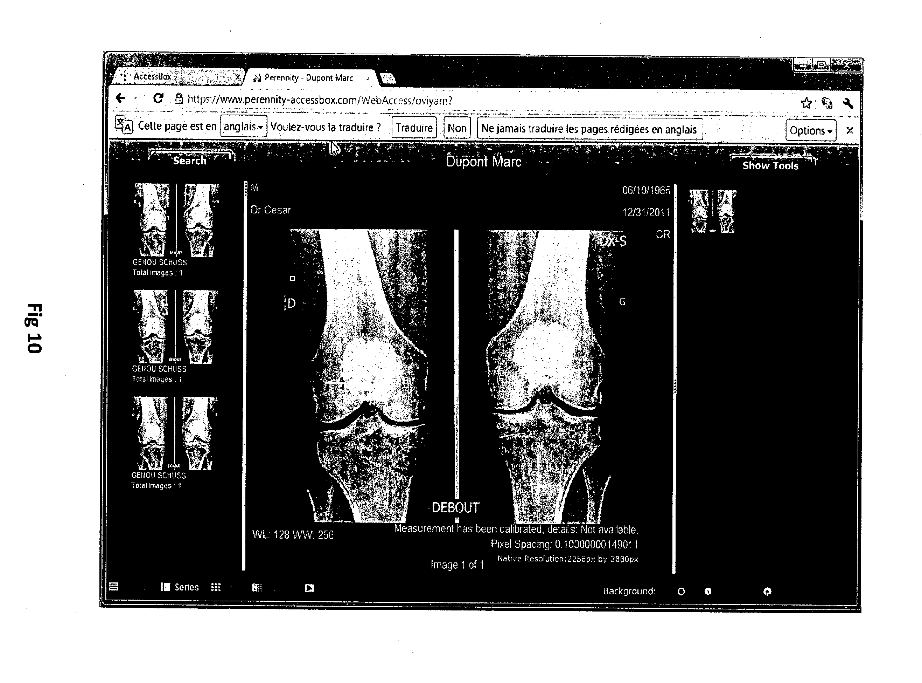

Reference is made to Figure 10 for illustrative purposes.

Access right management and Patient information protection are managed by two modules and technologies. First, the transfer of the data (i.e. the medical images) is encrypted using SSL (HTTPS) to avoid third parties having access to Patient information. Second, a dedicated module manages access rights depending on the profile of the user (a radiologist has access to all his images, a referring physician is limited to the images of his patients and patients have only access to their own images). While this access control is fully automated, it is possible to assign additional access right manually to extra users like specialists for a second opinion.

The names of the referring physician and patient are stored in the DICOM images as metadata. This information lets the WEB portal automatically assign access rights. In some cases, the name of either the physician or the patient is missing or is not correctly spelled. In that particular case, access rights must be managed manually.

Most of the large hospitals and modern clinics own a RIS (Radiology Information System) or a HIS (Hospital Information System) that are management applications to plan examinations including radiology. The name of a referring physician or a patient can also be retrieved from the RIS or the HIS using the embedded HL-7 server.

### - Web Portal Workflow

The Apparatus compresses medical images by series (i.e. images generated by one modality and belonging to one patient) in a Series container before sending it to Amazon S3 where they are securely stored. The WEB server used for our portal is hosted by a Windows 2008/Linux Amazon EC2 virtual server having a storage capacity of 100GB. We have developed a gateway to transfer images from the S3 storage pool to the EC2 web server. The gateway module scans the S3 and download then newly added ZIP containers that have been uploaded by an Apparatus. The gateway decompresses the images and sends them to a genuine DICOM server also hosted on the virtual WEB server. The images are the stored on the storage space of that server. When the disk space is almost full, the oldest images are automatically deleted to leave space for the newly added images (FIFO). The deleted images are still available from the S3 storage pool. As result, when a user is querying the WEB portal, he will have two options: if the images are still available on the EC2 server, they will be instantly shown. If the images are only available on the S3 platform, our gateway module will get the instruction to download those images from the S3 storage pool, decompress and store them on the EC2 server.

When images are available for a referring physician, he will be notified by email. This email contains a WEB link pointing directly to the newly added series of images belonging to that referring physician. Obviously, all users getting a link to a series of images must log in using their user name and password that have been provided beforehand by the clinic, radiologist or hospital.

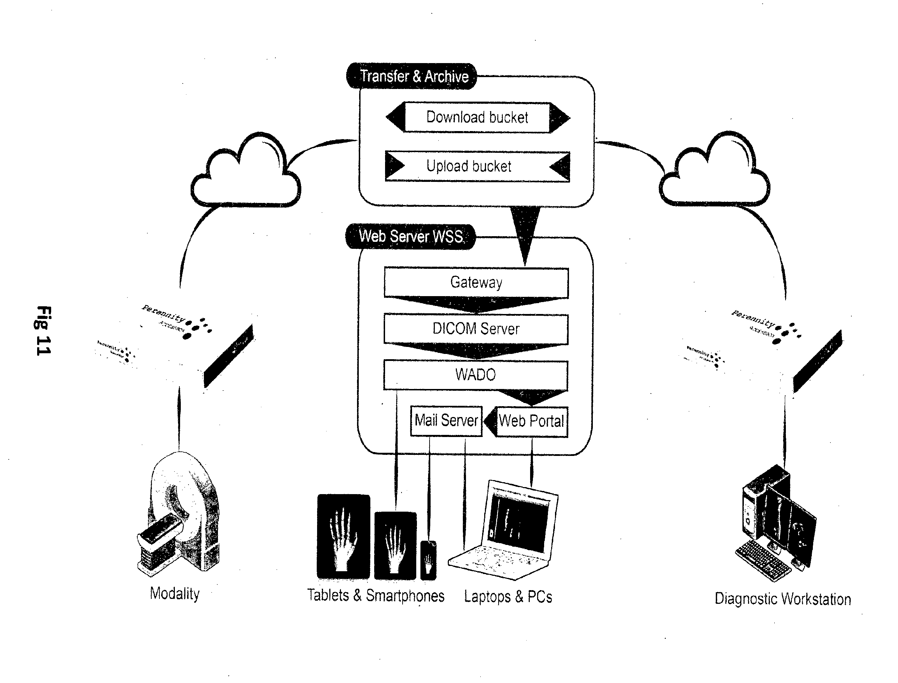

Reference is made to Figure 11 for illustrative purposes.
- Automated backup of the database and the configuration

According to a preferred embodiment, the Apparatus is equipped with an external, detachable data storage device, such as e.g. a USB key. This device is used to backup the configuration files and the database.

If the Apparatus is using Amazon S3 or a FTPS/SFTP server and/or a secondary storage device, an extra copy of both configuration and database will be stored on those storage platforms.

### - « Disaster recovery » - recovery of the database and parameters

The maintenance and repair procedures of the Apparatus are straightforward. In case of a failure (e.g. the hard drive is defective), a new Apparatus is sent to the end-user. He only needs to remove the external detachable data storage device such as e.g. the USB key from the defective Apparatus and insert it in the new one. When powering on the new Apparatus, it will automatically recover its configuration and database. If all images stored in an Apparatus are replicated to a local secondary storage, which is highly recommended, the data stored on the defective hard drive will also automatically be restored to the new Apparatus thanks to the database keeping track of the path of all files initially stored on the internal hard drive but having been replicated to the secondary storage.

In case of a disaster (fire, earthquake, ...), the Apparatus and the NAS system(s) being destroyed, it is still possible to recover all the data thanks to the copy of the images, configuration and database stored on Amazon S3 or a FTPS/SFTP server.

## Claims

1. Apparatus for receiving, storing, sharing and transmitting digital medical image data, comprising a processor, a hard drive, and connectivity means for communicating with a local or global network, **characterised in that** the processor comprises means for:
a. hosting a server supporting receipt, compression, encryption and data integrity check of the medical image data received from medical modalities;
b. storing of the medical image data on the hard drive of the apparatus;
c. providing access to the apparatus' web based administration and management interface;
d. supporting the query and retrieval of medical image data stored in the apparatus from any modality.

2. Apparatus according to claim 1, wherein the medical image data are formatted according to the DICOM standard

3. Apparatus according to any of the preceding claims, further comprising a detachable data storage unit, preferably a USB stick, for storing a software activation key, an encrypted copy of the configuration files/parameters , a daily backup of the local database and a network file template allowing to change the settings of the apparatus.

4. Apparatus according to any of the preceding claims, wherein the server further supports replicating the data stored on the hard drive to an external secure data-storage center.

5. Apparatus according to claim 4, wherein the server supporting replicating the data comprise pre-installed or pre-configured replication parameters.

6. Apparatus according to any of the preceding claims, further comprising a wireless communication module.

7. Apparatus according to claim 4 or 5, wherein the external secure data-storage center comprises a NAS, DAS or CAS.

8. Apparatus according to claim 8, wherein in case the apparatus is running out of space the server further supports the automatic deletion of the eldest images.

9. Apparatus according to any of the preceding claims, further comprising a router, preferably working in accordance with the DICOM standard, transmitting the data according to pre-defined criteria, and/or modifying the metadata comprised in the data according to pre-defined criteria.

10. Apparatus according to any of the preceding claims, wherein the means for compressing the medical image data comprises lossless compressing the data in ZIP files before transmission.

11. Apparatus according to any of the preceding claims, wherein the means for hosting the server performs the data integrity check on all data before transmission.

12. Apparatus according to any of the preceding claims, further comprising means for publishing the data to a WEB portal thereby providing access to and review of the data by referring physicians or patients.

13. Apparatus according to any of the preceding claims, wherein said apparatus is devoid of a graphical user interface and is not directly connected to a keyboard, a mouse, or a display.

14. Apparatus according to any of the preceding claims, wherein the processor further comprises means for automatically restoring in the event of an interruption of the communication between the apparatus and the local or global network the transmission of medical image data from the point of interruption.

15. Apparatus according to any of the preceding claims, wherein the data before transmission are encrypted by means of asymmetric cryptography and wherein the private key is generated by a random generator comprised in the processor.
